(19) Europäisches Patentamt
European Patent Office
Office européen des brevets

(11) Veröffentlichungsnummer: **0 025 119**
**B1**

(12) # EUROPÄISCHE PATENTSCHRIFT

(45) Veröffentlichungstag der Patentschrift: **25.05.83**

(51) Int. Cl.³: **C 07 D 239/26**, C 09 K 3/34

(21) Anmeldenummer: **80104661.6**

(22) Anmeldetag: **07.08.80**

(54) **Nematische kristallin-flüssige 5-Alkyl-2-(4-acyloxyphenyl)-pyrimidine für optoelektronische Anordnungen und Verfahren zu ihrer Herstellung.**

(30) Priorität: **20.08.79 DD 215057**
**20.08.79 DD 215058**

(43) Veröffentlichungstag der Anmeldung: **18.03.81 Patentblatt 81/11**

(45) Bekanntmachung des Hinweises auf die Patenterteilung: **25.05.83 Patentblatt 83/21**

(84) Benannte Vertragsstaaten: **CH DE FR GB LI**

(56) Entgegenhaltungen:
**DE-A-2 257 588**
**DE-A-2 547 737**
**DE-A-2 846 409**
**ZEITSCHRIFT FÜR CHEMIE, Band 15, Nr. 11 1975 H. ZASCHKE et al. »Synthese niedrigschmelzender kristallin-flüssiger Heterocyclen; 5-n-Alkyl-2-(4-n-alkanoyloxy-phenyl)-pyrimidine« Seiten 441 bis 443**

**Die Akte enthält technische Angaben, die nach dem Eingang der Anmeldung eingereicht wurden und die nicht in dieser Patentschrift enthalten sind.**

(73) Patentinhaber: **VEB Werk Für Fernsehelektronik im VEB Kombinat Mikroelektronik, 1160 Berlin-Oberschöneweide, Ostendstrasse 1-5 (DD)**

(72) Erfinder: **Demus, Dietrich, Dr. Dipl.-Chem., Veilchenweg 22, 4020 Halle (DD)**
Erfinder: **Zaschke, Horst, Dr. Dipl.-Chem., Händelstrasse 37, 4020 Halle (DD)**
Erfinder: **Wiegeleben, Albrecht, Dr. Dipl.-Chem., Goethestrasse 8, 4020 Halle (DD)**
Erfinder: **Böttger, Uta, Dipl.-Chem., Röpziger Strasse 10, 4020 Halle (DD)**

(74) Vertreter: **Braun, André et al, A. Braun, Braun, Héritier, Eschmann AG Patentanwälte Holbeinstrasse 36-38, CH-4051 Basel (CH)**

EP 0 025 119 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Nematische kristallin-flüssige 5-Alkyl-2-(4-acyloxyphenyl)-pyrimidine für optoelektronische Anordnungen und Verfahren zu ihrer Herstellung

Die Erfindung betrifft die Anwendung nematischer kristallin-flüssiger Substanzen in optoelektronischen Anordnungen zur Modulation des auffallenden oder durchgehenden Lichtes sowie zur Darstellung von Ziffern, Zeichen und Bildern.

Es ist bekannt, daß nematische flüssige Kristalle auf Grund ihrer dielektrischen Anisotropie in elektrischen Feldern verschiedene elektrooptische Effekte zeigen [G. Meier, E. Sackmann, J. G. Grabmaier, Application of Liquid Crystals, Berlin—Heidelberg—New York, Springer-Verlag 1975]. In geeigneten Zellen kann man mit Hilfe dieser Effekte eine Lichtmodulation erreichen sowie Ziffern, Zeichen und Bilder darstellen.

Nematische flüssige Kristalle mit negativer dielektrischer Anisotropie zeigen den dynamischen Streueffekt, der die Konstruktion von optoelektronischen Bauelementen ohne die Verwendung von Polarisatoren erlaubt. In nematischen flüssigen Kristallen mit positiver dielektrischer Anicotropie tritt der Schadt-Helfrich-Effekt auf, der auf der Deformation künstlich verdrillter nematischer Schichten in elektrischen Federn beruht. Diese Anordnung benötigt zwei Polarisationsfilter.

Bekannt ist des weiteren die Substanzklasse der 2-kernigen Derivate des Phenylpyrimidins, wie beispielsweise in der DE-A1-2 846 409 beschrieben. Sie zeichnet sich durch niedrige Klärpunkte aus, so daß sie bei ihrer Verwendung in Gemischen eine Klärpunkterniedrigung bewirkt. Diese Eigenschaft schränkt das Einsatzgebiet dieser flüssigkristallinen Substanzen ein.

Es gibt zur Zeit weder eine reine Verbindung noch Gemische mehrerer Substanzen, welche alle Anforderungen erfüllen, die an eine Substanz für optoelektronische Bauelemente gestellt werden, insbesondere niedrige Schmelz- und hohe Klärtemperaturen. Durch Zumischen weiterer Substanzen lassen sich die Eigenschaften von Gemischen verbessern, da im allgemeinen die Schmelztemperatur eutektischer Gemische mit der Zahl der Komponenten absinkt. Durch Einsatz von zusätzlichen Komponenten mit hohen Klärtemperaturen können die Klärpunkte von Gemischen bedeutend erhöht werden.

Ziel der Erfindung sind neue nematische flüssige Kristalle mit niedrigen Schmelz- und besonders hohen Klärpunkten für die Anwendung in optoelektronischen Bauelementen sowie Verfahren zu ihrer Herstellung.

Es wurde gefunden, daß kristallin-flüssige 5-Alkyl-2-(4-acyloxy-phenyl)-pyrimidine der allgemeinen Formel

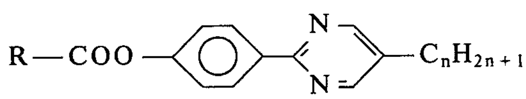

wobei

R = 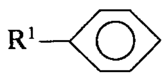 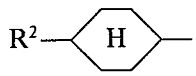

$R^1 = C_nH_{2n+1}, C_nH_{2n+1}O, C_nH_{2n+1}OCOO, C_nH_{2n+1}COO,$
$C_nH_{2n+1}S, F, Cl, Br, NO_2, CN, CF_3$

$R^2 = C_nH_{2n+1}$

mit n = 1 bis 10 bedeuten, hergestellt werden können durch Umsetzen von 4-Hydroxy-benzamidin-hydrochlorid mit α-Alkyl-β-dimethylaminoacrolein bei erhöhter Temperatur in Gegenwart von Natrium-methylat zu 5-Alkyl-2-[4-hydroxy-phenyl]-pyrimidin, worauf dieses mit einer Carbonsäure bzw. mit einem reaktionsfähigen Säurederivat, vorzugsweise einem Säurechlorid, verestert wird.

So kann beispielsweise 5-n-Hexyl-2-[4-(4-n-hexyl-benzoyloxy)-phenyl]-pyrimidin dadurch hergestellt werden, daß 4-Hydroxy-benzamidin-hydrochlorid mit α-n-Hexyl-β-dimenthylamino-acrolein bei erhöhter Temperatur von 310 bis 370°K in Gegenwart von Natriummethylat umgesetzt und das Reaktionsprodukt mit 4-n-Hexyl-benzoylchlorid verestert wird.

Erfindungsgemäß können

5-n-Pentyl-2-[4-(4-n-hexyl-benzoyloxy)-phenyl]-pyrimidin,
5-n-Hexyl-2-[4-(4-n-hexyl-benzoyloxy)-phenyl]-pyrimidin,
5-n-Heptyl-2-[4-(4-n-hexyl-benzoyloxy)-phenyl]-pyrimidin,
5-n-Octyl-2-[4-(4-n-hexyl-benzoyloxy)-phenyl]-pyrimidin,
5-n-Hexyl-2-[4-(4-methoxy-benzoyloxy)-phenyl]-pyrimidin,
5-n-Hexyl-2-[4-(4-n-propoxy-benzoyloxy)-phenyl]-pyrimidin,
5-n-Hexyl-2-[4-(4-n-heptyloxy-benzoyloxy)-phenyl]-pyrimidin,
5-n-Hexyl-2-[4-(4-methyl-benzoyloxy)-phenyl]-pyrimidin,

5-n-Hexyl-2-[4-(4-brom-benzoyloxy)-phenyl]-pyrimidin,
5-n-Hexyl-2-[4-(4-chlor-benzoyloxy)-phenyl]-pyrimidin,
5-n-Hexyl-2-[4-(5-fluor-benzoyloxy)-phenyl]-pyrimidin,
5-n-Hexyl-2-[4-(4-methylthio-benzoyloxy)-phenyl]-pyrimidin,
5-n-Hexyl-2-[4-(4-acetyloxy-benzoyloxy)-phenyl]-pyrimidin,
5-n-Hexyl-2-[4-(4-methoxycarbonyloxy-benzoyloxy)-phenyl]-pyrimidin,
5-n-Hexyl-2-[4-(4-trifluormethyl-benzoyloxy)-phenyl]-pyrimidin,
5-n-Heptyl-2-[4-(4-nitro-benzoyloxy)-phenyl]-pyrimidin,
5-n-Octyl-2-[4-(4-cyanbenzoyloxy)-phenyl]-pyrimidin,
5-n-Hexyl-2-[4-(4-n-propyloxy-benzoyloxy)-phenyl]-pyrimidin,
5-n-Hexyl-2-[4-(trans-4-methyl-cyclohexylcarbonyloxy)-phenyl]-pyrimidin,
5-n-Pentyl-2-[4-(4-n-pentyl-benzoyloxy)-phenyl]-pyrimidin,
5-n-Pentyl-2-[4-(trans-4-n-pentyl-cyclohexylcarbonyloxy)-phenyl]-pyrimidin

auf diese Weise hergestellt werden.

Erfindungsgemäß werden die 5-Alkyl-2-(4-acyloxy-phenyl)-pyrimidine für sich allein oder in Mischungen untereinander sowie vorzugsweise in Mischungen mit anderen kristallin-flüssigen oder nicht kristallin-flüssigen Komponenten vorteilhaft in optoelektronischen Bauelementen eingesetzt.

Eingesetzt wird erfindungsgemäß eine Mischung aus:

n-Propyl-cyclohexancarbonsäure-4-cyan-phenylester,
n-Butylcyclohexancarbonsäure-4-cyan-phenylester,
n-Pentylcyclohexancarbonsäure-4-cyanphenylester,
5-n-Hexyl-2-(4-n-hexylbenzoyloxyphenyl)-pyrimidine

Eine weitere Mischung besteht aus:

n-Propyl-cyclohexancarbonsäure-4-cyan-phenylester,
n-Butylcyclohexancarbonsäure-4-cyan-phenylester,
n-Pentylcyclohexancarbonsäure-4-cyan-phenylester,
5-n-Hexyl-2-(4-hexylbenzoyloxyphenyl)-pyrimidin.

Der Vorteil der erfindungsgemäßen eingesetzten Substanzen besteht darin, daß sie hohe Klärpunkte sowie in Gemischen hinreichend niedrige Schmelzp8nkte besitzen. Sie sind stabil gegenüber Luft, Wasser, erhöhter Temperatur und Lichteinwirkung, was sehr vorteilhaft für ihren Einsatz in LCD-Bauelementen ist.

Die Erfindung soll nachfolgend anhand von sechs Beispielen näher erläutert werden:

Beispiel 1

Beispiele für die entsprechend der Erfindung eingesetzten Substanzen sind in den folgenden Tabellen verzeichnet.

Tabelle 1

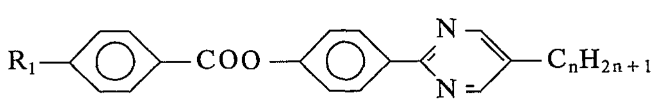

| N° | $R^1$ | n | K | Schmelzpkt. | S | M | Klärpkt. | $I_S$ |
|---|---|---|---|---|---|---|---|---|
| 1 | $C_6H_{18}$— | 5 | . | 57 | – | . | 157 | . |
| 2 | $C_6H_{13}$— | 6 | . | 43 | – | . | 147 | . |
| 3 | $C_6H_{13}$— | 7 | . | 64 | – | . | 150 | . |
| 4 | $C_6H_{13}$ | 8 | . | 75,5 | – | . | 142,5 | . |
| 5 | $CH_3$—O— | 6 | . | 116 | – | . | 196,5 | . |
| 6 | $CH_3H_7O$— | 6 | . | 103 | – | . | 187 | . |
| 7 | $C_7H_{15}O$— | 6 | . | 72,5 | – | . | 166 | . |

| N° | $R^1$ | n | K | Schmelzpkt. | S | | N | Klärpkt. | $I_S$ |
|---|---|---|---|---|---|---|---|---|---|
| 8 | $CH_3$— | 6 | . | 99 | – | | . | 116,5 | . |
| 9 | Br— | 6 | . | 120 | – | | . | 181 | . |
| 10 | Cl— | 6 | . | 118 | – | | . | 182 | . |
| 11 | F— | 6 | . | 116,5 | – | | . | 149 | . |
| 12 | $CH_3S$— | 6 | . | 110 | – | | . | 141 | . |
| 13 | $CH_3COO$— | 6 | . | 83 | – | | . | 195 | . |
| 14 | $CH_3OCOO$— | 6 | . | 88 | – | | . | 200 | . |
| 15 | $CF_3$ | 6 | . | 102 | . | 151 | . | 160,5 | . |
| 16 | $NO_2$ | 7 | . | 147 | . | 223 | . | 229 | . |
| 17 | CN | 8 | . | 144 | . | 159,5 | . | 240 | . |

Hierbei bedeuten:
K = kristallin-fest,
N = nematisch,
S = smektisch,
$I_S$ = isotrop-flüssig.

Tabelle 2

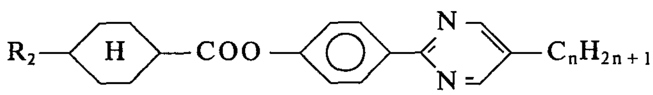

| $R^2$ | $C_nH_{2n+1}$ | K | Schmelz-pkt. | N | Klär-pkt. | $I_S$ |
|---|---|---|---|---|---|---|
| $CH_3$— | $C_6H_{13}$ | . | 98 | . | 141 | . |
| $C_5H_{11}$ | $C_5H_{11}$ | . | 97 | . | 185 | . |

## Beispiel 2

Eine Mischung aus drei Komponenten

| | |
|---|---|
| n-Propylcyclohexancarbonsäure-4-cyan-phenylester | 34,5 mol-% |
| n-Butylcyclohexancarbonsäure-4-cyan-phenylester | 31,0 mol-% |
| n-Pentylcyclohexancarbonsäure-4-cyan-phenylester | 34,5 mol-% |

bezeichnet als Mi 14, besitzt folgende Eigenschaften:

| | |
|---|---|
| Schmelzpunkt | +12,5 bis 16° C |
| Klärpunkt | 72° C |
| Schwellspannung | 1,3 V (f=500 hz, 25° C) |

Eine Mischung der Zusammensetzung

| | |
|---|---|
| Mi 14 | 80 mol-% |
| 5-n-Hexyl-2-(4-n-hexylbenzoyloxy-phenyl)-pyrimidin (N° 2 in Tab. 1) | 20 mol-% |

zeigt folgende Eigenschaften:

| | |
|---|---|
| Schmelzpunkt | +13,5 bis 25° C; stark unterkühlbar, bei −18° C Kristallisation erst nach sechs bis sieben Stunden |
| Klärpunkt | +94° C |
| Schwellspannung | 1,69 V (f=500 Hz, 24° C) |

Beispiel 3

Ein Gemisch aus

| | |
|---|---|
| Mi 14 | 90 mol-% |
| 5-n-Hexyl-2-(4-n-hexylbenzoyloxy-phenyl)-pyrimidin (N° 2 in Tab. 1) | 10 mol-% |

besitzt positive dielektrische Anisotropie und zeigt den Schadt-Helfrich-Effekt in verdrillten Schichten.

| | |
|---|---|
| Schmelzpunkt | +3,5 bis 9° C |
| Klärpunkt | 80 bis 81° C |
| Schwellspannung | 1,78 V (500 Hz bei 24° C) |

Das Gemisch ist stark unterkühlbar und kristallisiert bei −18° C erst nach mehreren Stunden.

Beispiel 4

Herstellung von 5-n-Alkyl-2-(4-hydroxy-phenyl)-pyrimidinen

Zu einer Natriummethylatlösung aus 9,2 g (0,4 g-Atome) Natrium in 80 ml abs. Methanol werden unter Rühren 17,4 g (0,1) Mol) 4-Hydroxy-benzamidinhydrochlorid und 0,1 Mol des entsprechenden $\alpha$-Alkyl-$\beta$-dimenthylamino-acroleins gegeben. Danach wird sechs Stunden unter Rückfluß gekocht. Die Reaktionsmischung wird am Rotationsverdampfer eingeengt, mit 100 ml verd. Essigsäure versetzt und die Lösung mehrmals mit Äther extrahiert. Der Ätherextrakt wird mit Wasser gewaschen, über $Na_2SO_4$ getrocknet, zur Trockne eingeengt und der Rückstand aus n-Hexan kristallisiert.

Tabelle 1

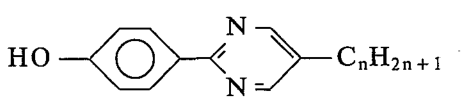

| n | Ausbeute % | F °C |
|---|---|---|
| 5 | 89 | 131 |
| 6 | 95 | 95 |
| 7 | 93 | 88 |
| 8 | 96 | 79 |

Beispiel 5

Herstellung der 5-n-Alkyl-2-[4-(4-subst-benzoyloxy)-phenyl]-pyrimidine

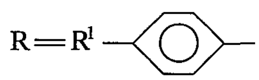

Zu einer Natriummethylatlösung von 0,23 g (0,01 Atome) Natrium in 30 ml abs. Methanol werden unter Rühren 0,01 Mol 3 und 0,02 Mol substituiertes Benzoylchlorid gegeben. Das Reaktionsgemisch wird zehn Stunden bei Raumtemperatur gerührt und über Nacht stehen gelassen. Nach Zugabe von Wasser wird ausgeäthert, der Ätherextrakt gewaschen, mit $Na_2SO_4$ und Aktivkohle behandelt und danach zur Trockne eingeengt.

Der Rückstand wird aus n-Hexan mehrmals umkristallisiert bzw. an einer $Al_2O_3$-Säule (Aktivitätsstufe 1) mit Äther oder Methylenchlorid chromatographiert und nach Entfernen des Lösungsmittels aus n-Hexan kristallisiert.

Tabelle 2

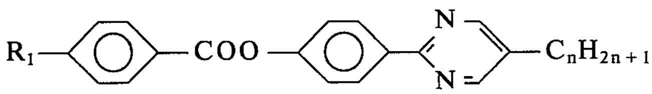

| $R^1$ | n | Ausb. % | K | Schmelz-pkt. | S | | N | Klär-pkt. | I |
|---|---|---|---|---|---|---|---|---|---|
| $C_6H_{13}$— | 5 | 73 | . | 57 | | | . | 157 | . |
| $C_6H_{13}$– | 6 | 70 | . | 43 | | | . | 147 | . |
| $C_6H_{13}$ | 7 | 95 | . | 64 | | | . | 150 | . |
| $C_6H_{13}$— | 8 | 78 | . | 75,5 | | | . | 142,5 | . |
| $CH_3O$— | 6 | 90 | . | 118 | | | . | 196,5 | . |
| $C_3H_7O$– | 6 | 81 | . | 103 | | | . | 187 | . |
| $C_7H_{15}O$— | 6 | 86 | . | 72,5 | | | . | 166 | . |
| $CH_3$— | 6 | 81 | . | 99 | | | . | 165 | . |
| Br— | 6 | 90 | . | 120 | | | . | 181 | . |
| Cl— | 6 | 88 | . | 118 | | | . | 182 | . |
| F— | 6 | 75 | . | 116,5 | | | . | 149 | . |
| $CH_3S$— | 6 | 68 | . | 110 | | | . | 141 | . |
| $CH_3COO$— | 6 | 65 | . | 83 | | | . | 195 | . |
| $CH_3OCOO$— | 6 | 55 | . | 88 | | | . | 200 | . |
| $CF_3$ | 6 | 68 | . | 102 | . | 151 | . | 160,5 | . |
| $NO_2$ | 7 | 78 | . | 147 | . | 223 | . | 229 | . |
| CN | 8 | 70 | . | 144 | . | 159,5 | . | 240 | . |

Hierbei bedeuten:
K = kristallin-fest,
N = nematisch,
I = isotrop-flüssig.

Umwandlungstemperaturen in °C.

Beispiel 6

Herstellung der 5-Alkyl-2-(4-(4-subst.-cyclohexyl-carbonyloxyphenyl)-pyrimidine

Die Synthese und Reinigung erfolgt analog Vorschrift 2 unter Einsatz von 4-subst.-Cyclohexancarbonsäurechloriden

Tabelle 3

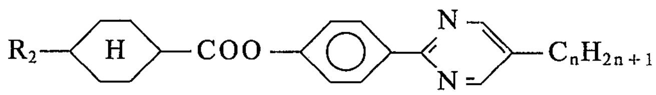

| $R^2$ | $C_nH_{2n+1}$ | Ausb. % | K | Schmelz-pkt. | N | Klärp. | I |
|---|---|---|---|---|---|---|---|
| $CH_3$— | $C_6H_{13}$ | 72 | . | 98 | . | 141 | . |
| $C_5H_{11}$— | $C_5H_{11}$— | 80 | . | 97 | . | 185 | . |

**Patentansprüche**

1. 5-Alkyl-2-(4-acyloxy-phenyl)-pyrimidine der allgemeinen Formel

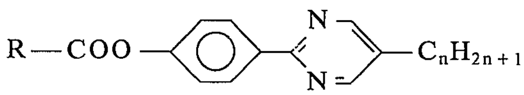

wobei

R = 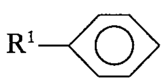 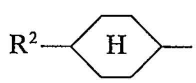

$R^1 = C_nH_{2n+1}, C_nH_{2n+1}O, C_nH_{2n+1}OCOO, C_nH_{2n+1}COO,$
$C_nH_{2n+1}S$, F, Cl, Br, $NO_2$, CN, $CF_3$

$R^2 = C_nH_{2n+1}$ mit n = 1 bis 10

bedeuten.

2. 5-n-Pentyl-2-[4-(4-n-hexyl-benzoyloxy)-phenyl]-pyrimidin.
3. 5-n-Hexyl-2-[4-(4-n-hexyl-benzoyloxy)-phenyl]-pyrimidin.
4. 5-n-Heptyl-2-[4-(4-n-hexyl-benzoyloxy)-phenyl]-pyrimidin.
5. 5-n-Octyl-2-[4-(3-n-hexyl-benzoyloxy)-phenyl]-pyrimidin.
6. 5-n-Hexyl-2-[4-(4-methoxy-benzoyloxy)-phenyl]-pyrimidin.
7. 5-n-Hexyl-2-[4-(4-n-propoxy-benzoyloxy)-phenyl]-pyrimidin.
8. 5-n-Hexyl-2-[4-(4-n-heptyloxy-benzoyloxy)-phenyl]-pyrimidin.
9. 5-n-Hexyl-2-[4-(4-methyl-benzoyloxy)-phenyl]-pyrimidin.
10. 5-n-Hexyl-2-[-(4-brom-benzoyloxy)-phenyl]-pyrimidin.
11. 5-n-Hexyl-2-[4-(4-chlor-benzoyloxy)-phenyl]-pyrimidin.
12. 5-n-Hexyl-2-[4-(5-fluor-benzoyloxy)-phenyl]-pyrimidin.
13. 5-n-Hexyl-2-[4-(4-methylthio-benzoyloxy)-phenyl]-pyrimidin.
14. 5-n-Hexyl-2-[4-(4-acetyloxy-benzoyloxy)-phenyl]-pyrimidin.
15. 5-n-Hexyl-2-[4-(4-methoxycarbonyloxy-benzoyloxy)-phenyl]-pyrimidin.
16. 5-n-Hexyl-2-[4-(4-trifluormethyl-benzoyloxy)-phenyl]-pyrimidin.
17. 5-n-Heptyl-2-[4-(4-nitro-benzoyloxy)-phenyl]-pyrimidin.
18. 5-n-Octyl-2-[4-(4-cyan-benzoyloxy)-phenyl]-pyrimidin.
19. 5-n-Hexyl-2-[4-(4-n-propyloxy-benzoyloxy)-phenyl]-pyrimidin.
20. 5-n-Hexyl-2-[4-(trans-4-methyl-cyclohexylcarbonyloxy)-phenyl]-pyrimidin.
21. 5-n-Pentyl-2-[4-(4-n-pentyl-benzoyloxy)-phenyl]-pyrimidin.

22. 5-n-Pentyl-2-[4-(trans-4-n-pentyl-cyclohexylcarbonyloxy)-phenyl]-pyrimidin.

23. Verfahren zur Herstellung kristallin-flüssiger 5-Alkyl-2-(4-acyloxy-phenyl)-pyrimidine nach Anspruch 1 bis Anspruch 22, dadurch gekennzeichnet, daß 4-Hydroxy-benzamidin-hydrochlorid mit $\alpha$-Alkyl-$\beta$-dimethylamino-acrolein bei erhöhter Temperatur in Gegenwart von Natriummethylat zu einem 5-Alkyl-2-(4-hydroxy-phenyl)-pyrimidin umgesetzt und dieses mit einem reaktionsfähigen Säurederivat, vorzugsweise einem Säurechlorid, verestert wird.

24. Verfahren nach Anspruch 23 zur Herstellung der Verbindung nach Anspruch 3, dadurch gekennzeichnet, daß 4-Hydroxybenzamidin-hydrochlorid mit $\alpha$-n-Hexyl-$\beta$-dimethylamino-acrolein bei 310 bis 370°K in Gegenwart von Natriummethylat umgesetzt und das Reaktionsprodukt mit 4-n-Hexyl-benzoylchlorid verestert wird.

25. Anwendung nematischer kristallin-flüssiger Substanzen, dadurch gekennzeichnet, daß kristallin-flüssige 5-Alkyl-2-(4-acyloxyphenyl)-pyrimidine nach Anspruch 1 bis 22 für sich allein oder in Mischungen untereinander sowie vorzugsweise in Mischungen mit anderen kristallin-flüssigen oder nicht kristallinflüssigen Komponenten in optoelektronischen Bauelementen eingesetzt werden.

26. Anwendung nematischer kristallin-flüssiger Substanzen nach Anspruch 25, dadurch gekennzeichnet, daß eine Mischung aus n-Propyl-cyclohexancarbonsäure-4-cyan-phenylester, n-Butylcyclohexancarbonsäure-4-cyan-phenylester, n-Pentylcyclohexancarbonsäure-4-cyan-phenylester, 5-n-Hexyl-2-(4-n-hexylbenzoyloxy-phenyl)-pyrimidin eingesetzt wird.

27. Anwendung nematischer kristallin-flüssiger Substanzen nach Anspruch 25, dadurch gekennzeichnet, daß eine Mischung aus n-Propylcyclohexancarbonsäure-4-cyan-phenylester, n-Butylcyclohexancarbonsäure-4-cyan-phenylester, 5-n-Hexyl-2-(4-hexylbenzoyloxy-phenyl)-pyrimidin eingesetzt wird.

## Claims

1. 5-Alkyl-2-(4-acyloxy-phenyl)-pyrimidines having the general formula:

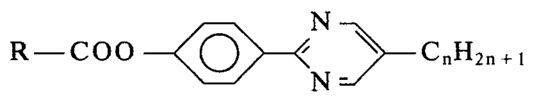

wherein

R = 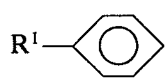 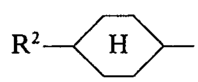

$R^1 = C_nH_{2n+1}, C_nH_{2n+1}O, C_nH_{2n+1}OCOO, C_nH_{2n+1}COO, C_nH_{2n+1}S, F, Cl, Br, NO_2, CN, CF_3$

$R^2 = C_nH_{2n+1}$ with $n = 1$ to 10.

2. 5-n-Pentyl-2-[4-(4-n-hexyl-benzoyloxy)-phenyl]-pyrimidine.
3. 5-n-Hexyl-2-[4-(4-n-hexyl-benzoyloxy)-phenyl]-pyrimidine.
4. 5-n-Heptyl-2-[4-(4-n-hexyl-benzoyloxy)-phenyl]-pyrimidine.
5. 5-n-Octyl-2-[4-(4-n-hexyl-benzoyloxy)-phenyl]-pyrimidine.
6. 5-n-Hexyl-2-[4-(4-methoxy-benzoyloxy)-phenyl]-pyrimidine.
7. 5-n-Hexyl-2-[4-(4-n-propoxy-benzoyloxy)-phenyl]-pyrimidine.
8. 5-n-Hexyl-2-[4-(4-n-heptyloxy-benzoyloxy)-phenyl]-pyrimidine.
9. 5-n-Hexyl-2-[4-(4-methyl-benzoyloxy)-phenyl]-pyrimidine.
10. 5-n-Hexyl-2-[4-(4-bromo-benzoyloxy)-phenyl]-pyrimidine.
11. 5-n-Hexyl-2-[4-(4-chloro-benzoyloxy)-phenyl]-pyrimidine.
12. 5-n-Hexyl-2-[4-(5-fluoro-benzoyloxy)-phenyl]-pyrimidine.
13. 5-n-Hexyl-2-[4-(4-methylthio-benzoyloxy)-phenyl]-pyrimidine.
14. 5-n-Hexyl-2-[4-(4-acetyloxy-benzoyloxy)-phenyl]-pyrimidine.
15. 5-n-Hexyl-2-[4-(4-methoxycarbonyloxy-benzoyloxy)-phenyl]-pyrimidine.
16. 5-n-Hexyl-2-[4-(4-trifluoromethyl-benzoyloxy)-phenyl]-pyrimidine.
17. 5-n-Heptyl-2-[4-(4-nitro-benzoyloxy)-phenyl]-pyrimidine.
18. 5-n-Octyl-2-[4-(4-cyano-benzolyoxy)-phenyl]-pyrimidine.
19. 5-n-Hexyl-2-[4-(4-n-propyloxy-benzoyloxy)-phenyl]-pyrimidine.
20. 5-n-Hexyl-2-[4-(trans-4-methyl-cyclohexylcarbonyloxy)-phenyl]-pyrimidine.
21. 5-n-Pentyl-2-[4-(4-n-pentyl-benzoyloxy)-phenyl]-pyrimidine.
22. 5-n-Pentyl-2-[4-(trans-4-n-pentyl-cyclohexylcarbonyloxy)-phenyl]-pyrimidine.
23. Process for the preparation of crystalline-liquid 5-alkyl-2-(4-acyloxy-phenyl)-pyrimidines

according to claim 1 to claim 22, characterized in that 4-hydroxybenzamidine-hydrochloride is reacted with α-alkyl-β-dimethylamino-acrolein at an elevated temperature in the presence of sodium methoxide to form a 5-alkyl-2-(4-hydroxy-phenyl)-pyrimidine and the latter is esterified with a reactive acid derivative, preferably an acid chloride.

24. Process according to claim 23 for the preparation of the compound according to claim 3, characterized in that 4-hydroxy-benzamidine-hydrochloride is reacted with α-n-hexyl-β-dimethylamino-acrolein at 310 to 370° K in the presence of sodium methoxide and the reaction product is esterified with 4-n-hexyl-benzoyl chloride.

25. Use of nematic crystalline-liquid substances, characterized in that crystalline-liquid 5-alkyl-2-(4-acyloxy-phenyl)-pyrimidines according to claim 1 to 22 are employed, alone or in mixtures with each other, and preferably in mixtures with other crystalline-liquid or non-crystalline-liquid components in opto-electronic arrangements.

26. Use of nematic crystalline-liquid substances according to claim 25, characterized in that a mixture of 4-cyano-phenyl n-propyl-cyclohexanecarboxylate, 4-cyano-phenyl n-butyl-cyclohexanecarboxylate, 4-cyano-phenyl n-pentyl-cyclohexanecarboxylate, 5-n-hexyl-2-(4-n-hexylbenzoyloxy-phenyl)-pyrimidine is employed.

27. Use of nematic crystalline-liquid substances according to claim 25, characterized in that a mixture of 4-cyano-phenyl n-propyl-cyclohexanecarboxylate, 4-cyano-phenyl n-butyl-cyclohexanecarboxylate, 5-n-hexyl-2-(4-hexyl-benzoyloxy-phenyl)-pyrimidine is employed.

**Revendications**

1. 5-Alkyl-2-(4-acyloxy-phényl)-pyrimidines, de formule générale

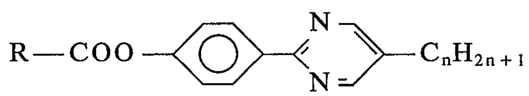

dans laquelle

R = 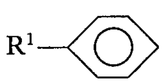 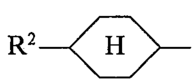

$R^1 = C_nH_{2n+1}, C_nH_{2n+1}O, C_nH_{2n+1}OCOO, C_nH_{2n+1}COO, C_nH_{2n+1}S, F, Cl, Br, NO_2, CN, CF_3$

$R^2 = C_nH_{2n+1}$, n = 1 à 10.

2. 5-n-Pentyl-2-[4-(4-n-hexyl-benzoyloxy)phényl]-pyrimidine.
3. 5-n-Hexyl-2-[4-(4-n-hexyl-benzoyloxy)phényl]-pyrimidine.
4. 5-n-Heptyl-2-[4-(4-n-hexyl-benzoyloxy)-phényl]-pyrimidine.
5. 5-n-Octyl-2-[4-(4-n-hexyl-benzoyloxy)-phényl]-pyrimidine.
6. 5-n-Hexyl-2-[4-(4-méthoxy-benzoyloxy)-phényl]-pyrimidine.
7. 5-n-Hexyl-2-[4-(4-n-propoxy-benzoyloxy)-phényl]-pyrimidine.
8. 5-n-Hexyl-2-[4-(4-n-heptyloxy-benzoyloxy)-phényl]-pyrimidine.
9. 5-n-Hexyl-2-[4-(4-méthyl-benzoyloxy)-phényl]-pyrimidine.
10. 5-n-Hexyl-2-[4-(4-bromo-benzoyloxy)-phényl]-pyrimidine.
11. 5-n-Hexyl-2-[4-(4-chloro-benzoyloxy)-phényl]-pyrimidine.
12. 5-n-Hexyl-2-[4-(5-fluoro-benzoyloxy)-phényl]-pyrimidine.
13. 5-n-Hexyl-2-[4-(4-méthylthio-benzoyloxy)-phényl]-pyrimidine.
14. 5-n-Hexyl-2-[4-(4-acétyloxy-benzoyloxy)-phényl]-pyrimidine.
15. 5-n-Hexyl-2-[4-(4-méthoxycarbonyloxy-benzoyloxy)-phényl]-pyrimidine.
16. 5-n-Hexyl-2-[4-(4-trifluorométhyl-benzoyloxy)-phényl]-pyrimidine.
17. 5-n-Heptyl-2-[4-(4-nitro-benzoyloxy)-phényl]-pyrimidine.
18. 5-n-Octyl-2-[4-(4-cyano-benzoyloxy)-phényl]-pyrimidine.
19. 5-n-Hexyl-2-[4-(4-propyloxy-benzoyloxy)-phényl]-pyrimidine.
20. 5-n-Hexyl-2-[4-(trans-4-méthyl-cyclohexyl-carbonyloxy)-phényl]-pyrimidine.
21. 5-n-Pentyl-2-[4-(4-n-pentyl-benzoyloxy)-phényl]-pyrimidine.
22. 5-n-Pentyl-2-[4-(trans-4-n-pentyl-cyclohexyl-carbonyloxy)-phényl]-pyrimidine.
23. Procédé de préparation de cristaux liquides de 5-alkyl-2-(4-acyloxy-phényl)-pyrimidines selon les

revendications 1 à 22, caractérisé en ce que l'on fait réagir le chlorhydrate de 4-hydroxy-benzamidine avec une α-alkyl-β-diméthylamino-acroléine à température élevée en présence de méthylate de sodium pour former une 5-alkyl-2-(4-hydroxy-phényl)-pyrimidine et l'on estérifie ce composé avec un dérivé réactif d'acide, de préférence un chlorure d'acide.

24. Procédé de préparation selon la revendication 23, du composé selon la revendication 3, caractérisé en ce que l'on fait réagir le chlorhydrate de 4-hydroxy-benzamidine avec l'α-n-hexyl-β-diméthylamino-acroléine à une température de 310 à 370° K en présence de méthylate de sodium et esterifie le produit de la réaction par le chlorure de 4-n-hexyl benzoyle.

25. Utilisation de cristaux liquides nématiques, caractérisée en ce que l'on utilise des cristaux liquides de 5-alkyl-2-(4-acyloxy-phényl)-pyrimidines selon les revendications 1 à 22, seuls ou mélangés les uns avec les autres ainsi que de préférence en mélange avec d'autres cristaux liquides ou des constituants autres que des cristaux liquides dans des dispositifs opto-électroniques.

26. Utilisation de cristaux liquides nématiques selon la revendication 25, caractérisée en ce que l'on utilise un mélange de n-propyl-cyclohexanecarboxylate de 4-cyano-phényle, de n-butylcyclohexane-carboxylate de 4-cyano-phényle, de n-pentylcyclohexanecarboxylate de 4-cyano-phényle et de 5-n-hexyl-2-(4-n-hexylbenzoyloxy-phényl)-pyrimidine.

27. Utilisation de cristaux liquides nématiques selon la revendication 25, caractérisée en ce que l'on utilise un mélange de n-propyl-cyclohexanecarboxylate de 4-cyanophényle, de n-butylcyclohexane-carboxylate de 4-cyanophényle et de 5-n-hexyl-2-(4-hexylbenzoyloxy-phényl)-pyrimidine.